# EUROPEAN PATENT SPECIFICATION

(11) **EP 2 475 312 B1**
(45) Date of publication and mention of the grant of the patent: **18.05.2016**
(21) Application number: 10726301.4
(22) Date of filing: 09.06.2010
(51) Int. Cl.: A61B 17/34

(54) **SURGICAL ACCESS DEVICE**
CHIRURGISCHER ZUGANG
INSTRUMENT CHIRURGICAL D'ACCÈS

(30) Priority: 20.07.2009 US 226779 P
(43) Date of publication of application: 18.07.2012
(73) Proprietor: THE ADELMAN RESEARCH LTD., Victoria, Mahé (SC)
(72) Inventor: MEHDIZADE, Amir, 1205 Geneva (CH); EHTESHAMI, John Reza, Phoenix, Arizona 85020 (US)
(74) Representative: reuteler & cie SA
(86) International application number: PCT/IB2010/052570
(87) International publication number: WO 2011/010229

(56) References cited:
- EP-A2- 0 872 215
- WO-A1-2008/066557
- WO-A1-2008/097665
- GB-A- 2 437 159
- US-A- 5 390 671
- US-A- 5 730 754
- US-A1- 2005 288 550
- US-A1- 2008 132 764

## Description

### Field of invention

The present invention relates to a surgical device for operating or gaining access to tissue for diagnostic or therapeutic purposes. The present invention relates in particular, but not exclusively, to surgical devices for operating or gaining access to hard tissues such as bone tissue, or soft tissues such as cartilage, ligaments, and tendons.

### Background

Spinal stenosis is a condition characterized by a narrowing of the central canal. Severe cases of spinal stenosis require decompressive surgery by removing the hypertrophied bone and ligaments that are causing the compression. Posterior spinal surgeries for stenosis can be performed by indirectly enlargening the central canal by reshaping the posterior vertebral skeletal structures. Surgical methods to create additional room in the central canal include laminotomy and laminoplasty. In laminoplasty the lamina on one side are divided, while the lamina on the contra lateral side is partially divided and hinged. A spacer or other fixation device is placed on the lamina on the cut side to widen the central canal. Alternatively laminoplasty can also be performed by splitting the spinous process and partially cutting each lamina on both sides and hinging the lamina bilaterally with a spacer in between the divided ends of the spinous process. Decompressive surgery such as laminectomy, laminotomy, and laminoplasty are standard procedures for patients with spinal stenosis, when non-surgical treatments have failed. In certain conventional procedures, a large straight cannula is used for transient access to the area undergoing surgical intervention. Such conventional cannulas are non-powered hand instruments that create a portal large enough to allow operating access of surgical instruments to the surgical area and a direct visual view of the surgical area during operation for the surgeon. These decompressive surgeries are open surgical procedures, resulting in extensive recovery times for patients, as well as high costs, associated risks and complications.

Surgical procedures invariably involve trauma to tissues around the surgical site. Smaller skin incision and decreased disruption of surrounding muscle tissue during the surgical approach reduces postsurgical healing time, and pain. Thus, there is increasing demand for minimally invasive surgical (MIS) techniques to perform surgical procedures, through smaller incisions with less disruption of the surrounding soft tissues, with the goal of decreased recovery times, lessened morbidity, and cost savings.

US2007270866A and WO 2008/097665 disclose prior examples of spinal access devices for performing spinal surgery on a patient.

The invention is defined in claim 1. Preferred embodiments are recited in the dependent claims.

### Summary of the invention

An object of the invention is to provide a surgical device that is minimally invasive, safe to use and that reduces operation times and patient recovery.
It is advantageous to provide a surgical device that is versatile and able to perform different surgical steps or operations.
A particular object of the invention is to provide a surgical device for operations on the vertebrae, such as laminectomy, laminotomy, or laminoplasty that is minimally invasive and safe to use.
Disclosed herein is a surgical device for minimal invasive surgery (MIS), comprising a cannula with a wall surrounding at least one lumen extending from an access end configured to remain outside of a patient's body, to a tissue tip which is configured to be inserted into the patient and to rests over or against the treatment area. The tissue tip imay be configured in different shapes to fit on to the tissue being treated. The edges of the tissue tip rest on the tissue adjacent to the area being treated. Additionally, one or more surgical instruments, or implantable medical devices are able to be received, to slide, and to be guided by the lumen of the cannula, wherein the surgical device in an embodiment, is configured to be used with a medical imaging system for image guidance of the cannula and surgical instruments and implantable medical devices used with the cannula.

The cannula's wall is configured to allow partial transparency with respect to a scanning signal of said medical imaging system such that at least portions of the instrument received in the cannula are visible. The thickness of the cannula wall in at least one scanning direction, in units of centimeters multiplied by the device material's density in units of grams per cubic centimeter, preferably ranges in value between 0.1 and 0.4 grams per centimeter squared to enable the partial transparency, while maintaining structural integrity. This range may be empirically determined based on the range of material density of the cannula, tested for transparency with imaging systems. Specific tests with CT and fluoroscopy were used with a variety of materials from peek having the lowest density at 1.3g/cc to 8g/cc for cobalt chrome and stainless steel being the highest density, and with the average approximating titanium and titanium alloys having a density of approximately 4.5g/cc. Given the forces exerted on the cannula as it is advanced through the surrounding tissue, the wall thickness of the cannula can range advantageously from approximately 1.5mm for peek to 0.5mm for cobalt chrome. By multiplying the density of the material in grams per cubic centimeters by the wall thickness in centimeters of the cannula that had good transparency during testing, the range between 0.1 and 0.4grams per centimeter squared was obtained. The operator may further choose cannulas made from different materials based on the type of image guidance system being used, since each material generates different imaging artifacts based on the imaging modality that is used.

In an embodiment of the present invention, the cross sectional outer dimensions of the cannula of the present invention may advantageously be less than 12mm in both height and width. The cross section is able to be made substantially smaller than the prior art, because the surgical instruments perform their function over the treatment area as they are advanced along the longitudinal axis of the cannula. This is different than the surgical instruments that are used with the prior art cannula which have to moved from one side of the cannula to the other side in the plane that is substantially perpendicular to the longitudinal axis and parallel to the cross section of the cannula. Therefore the cross section of the cannula of the present embodiment can perform the same task with a smaller lumen, since the cannula needs to only be large enough to allow passage of the surgical instruments, and medical devices through its lumen. In this embodiment of the present invention, the surgical instruments or medical devices used can be made to less than 10mm in diameter, therefore the lumen of the cannula needs to be only large enough to allow sliding of the surgical instruments through it. Given additional room for clearance for the surgical instrument or medical devices and the cannula's shell thickness the outer dimension of the cannula can be made 12mm or less, which is substantially smaller than the 16mm to 18 mm corresponding to the smallest diameter of prior art cannula. Even small changes in the diameter of the cannula can have substantial impact on the degree of trauma to the surrounding tissues as the cannula is inserted into the body of a patient, since the cross section of the cannula is determined by the square of the radius.

Also disclosed herein is a surgical device for invasive surgery, comprising of a cannula with a wall surrounding at least one lumen extending from an access end to a tissue tip, and one or more instruments receivable in the lumen of the cannula. In an embodiment the cannula has a non-straight shape with one, two, or more bends or curves to provide a short path to the surgical site while enabling optimal positioning of the tissue tip and surgical instrument in relation to the tissue structure to be operated. The cannula may be bent or curved essentially in a single plane, or bent or curved in at least two planes, especially for adapting to left or right side anatomical tissue structures. This curved configuration provides substantial advantages in terms of allowing the axis of the lumen of the cannula to approch the tissue being treated in an axis parrallel to the axis in which treatment is being carried out. Therefore the longitudinal axis of the surgical instruments or medical devices inserted through the lumen at the tissue tip will be parallel to the the direction in which the treatment will be performed, while the axis of the lumen at the access end will in a different plane. This advantageous configuration permits less distortion of the tissue surrounding the treatment area.

The cannula tissue tip is preferably configured to rest over, on or against the tissue to be operated, the tissue tip having an edge, and optionally specific right and left edges, shaped to conform to the shape of the tissue against which the tip is intended to rest. The edges of the tissue tip that are placed on the tissue adjacent to the tissue being treated may be positioned at the apex of the entry tip of the cannula, or be positioned at a specific distance behind the apex at the entry tip of the cannula. For better conformity to left and right sided tissue structures, the edge of the tissue tip may be non-symmetrical, with different size openings for the left and right side of the device's tissue tip. These openings may vary in both the longitudinal plane as well as the axial plane, to allow the edges of the functional tip to better conform to different length and height tissues.

Guide elements in the form of one or more railing protrusions or grooves or slits may be provided longitudinally, along the inner cannula wall to aid in guiding the instrument as it is received slides through the lumen of the cannula. Alternatively, the guiding elements may be configured to be placed on the surgical device itself, and allow receiving, sliding and guidance after insertion of the instrument. Depending on the surgical tool the instrument guide elements may comprise a bearing portion to support a rotating instrument.

The surgical device according to an embodiment of the invention is in particular configured for operation on the verterbrae, including cutting and burring operations of the vertebral lamina, and other segments of the posterior vertebral elements.

The present invention relates to the use of a novel straight, curved, or angled cannula with a novel tip that conforms substantially to the tissue that is being treated, and to the surrounding tissues. The cannula creates an access portal to the tissue structure being treated that is advantageous in the direction of access, minimization of dissection to the surrounding tissues, and control of instruments and devices inserted through the cannula. Additionally this novel cannula has a further unique feature that permits accurate and precise positioning of instruments and devices by use of an internal guiding mechanism that creates an instrument guide element between the instrument or medical device and the cannula. A further advantage of the cannula is the specific application of material properties and cannula geometry to minimize the artifact created by imaging systems used to visualize the treatment area when the cannula is inserted in that area. These advantages when compared to the prior art result in: increased safety for patients, decreased risk of complications associated with large surgical dissections, less pain, and ease of use for the operator.

Still, more particularly, the present invention relates to an MIS device for accessing the posterior vertebral structures. More specifically, we describe an MIS device that can be used if chosen in conjunction with visual or image assistance devices such as cameras, fluoroscopy, CT, MRI, flat panel, ultrasound, or computer assisted navigation. Advantageously the current invention overcomes the problem of extensive tissue dissection of open surgeries, and the moderate tissue dissection of current MIS devices. Further the struggle in current MIS devices to visualize the surgical field and identifying anatomical landmarks is overcome by the novel use of material properties and dimensions which permit less artifact during imaging while the cannula is in place over the treatment area. The use of smaller curved or angled cannula of an embodiment of the invention permits the user to approach the tissue structures being treated from more advantageous directions that are not possible using the conventional straight cannula.

One of the advantages of an embodiment of the invention is to introduce instruments or implantable medical devices approximately parallel to the spinal lamina or other skeletal structure being treated, with decreased trauma to the surrounding tissues. Current MIS techniques limit the operator to a relatively small range of directions or angles for approaching the surgical site. Embodiments of the current invention allow the operator more trajectories for accessing the treatment site. There has been no description of curved or angled cannula in the prior art in this field.

A further advantage is that a smaller cannula can be used if the longitudinal access of the cannula is significantly parallel to the tissue axis of the treatment being performed. For example, a burr can remove tissue as it is being advanced through the longitudinal access of the cannula in an embodiment of the invention. This provides adequate room in the lumen of the cannula for the cutting device to pass through. The cannula needs to only have an inside diameter that can permit the surgical instrument or medical device to pass through, rather than in the prior art where the burr or other device needs be moved across the cross section of the cannula. In the prior art the cannula diameter needs to be larger to provide greater exposure, to allow the operator an adequate visualization field, as well as having enough space to move the burr laterally inside the cannula. Therefore in the prior art, the treatment area is limited by the cannula's cross sectional area. Secondly, the diameter of the cannula needs to be larger due to the need for direct visualization of the tissue being treated.

A further advantage of the cannula of the present invention is a tissue tip significantly conforming to the tissue structure that is being treated. Also, the guiding track optional feature within the cannula's lumen permits the cannula to keep instruments and devices in a controlled position with respect to the cannula allowing the operator to perform the desired functions with greater control. Additionally when used in conjunction with the tissue tip of the cannula, which provides the device with substantial stability relative to the tissue being treated, instruments and devices can be introduced and to be used with greater precision and accuracy, in uniquely advantageous directions not possible with the cannula of the prior art.

The tissue tips with various sizes and designs can fit over posterior spinal elements such as the lamina, spinous process, facet joints, anterior structures such as the vertebral bodies, or other parts of the skeletal system. Furthermore, the tissue tip of the cannula in an embodiment of the invention has specific left and right sided designs, with asymmetrical contours and profiles on the left and right sided openings of tissue tip to conform to the asymmetry of the tissues being treated as well as surrounding tissues.

The tissue tip can partially, minimally or significantly conform to the structure being treated. This ability is advantageous in providing a greater degree of stability to the cannula, and permiting optimum positioning of the tissue tip with greater control of the treatment being performed. In the prior art the cannula maintains the same general tip design independent of the structure that is being treated, or which side of the body left or right that is being used on. The cannula tip may be designed to substantially accommodate the three dimensional geometry of the tissue to be treated. In an advantageous embodiment of the invention, the cannula tissue tip or shaft can rest against the adjacent skeletal structures and act as a fulcrum and a means of controlling the instruments being introduced for performing the procedure, or moving and reshaping cut segments of bone.

The present invention permits the user to perform procedures through a smaller skin incision than any of the prior art, causing decreased trauma to the surrounding structures, as well as taking a safer anatomical path to the treatment area. The devise of an embodiment of the invention allows the operator the ability to address specific areas of pathology with great precision and accuracy and when combined with image or other guiding systems, and provides unique advantages not present in prior systems.

The surgical device may be used with CT, MRI, fluoroscopic, navigation, flat panel, ultrasound or other imaging systems to gain access to the treatment area, and to perform the treatment without direct visualization of the tissue being treated. The cannula diameter can be made smaller since it only needs to accommodate the surgical instruments and devices. This overcomes the current limitation of existing MIS surgical methods and devices that use larger cannulas that require larger skin incisions to allow the operator to directly, or with use of cameras see the area being treated.

The surgical device according to an embodiment of the invention may be used to introduce instruments for cutting, decorticating, shaving, or deriding of bone and tissues attached to bone.

The surgical device according to an embodiment of the invention may be used to introduce bone graft, proteins, osteoinductive, or osteoconductive material through the device to the surgical site.

### Brief Description of the Drawings

Further objects, features and advantages of the invention will become apparent from the following detailed description taken in conjunction with the accompanying figures showing illustrative embodiments of the invention in which:
FIG. 1 shows the posterior view of the cervical spine with labeling of the anatomical sites;
FIG. 2 shows a lateral view of the cervical spine with labeling of the anatomical sites, axis lines parallel and perpendicular to the lamina, and axis line perpendicular to the skin;
FIG. 3 shows the axial view of the cervical spine with labeling of the anatomical sites, axis line perpendicular to the lamina in the transverse plane, and axis line perpendicular to the skin;
FIG. 4 shows the posterior view of the lumbar spine with labeling of the anatomical sites;
FIG. 5 shows lateral view of the lumbar spine with labeling of the anatomical sites, axis lines parallel and perpendicular to the lamina, and axis line perpendicular to the skin;
FIG 6 shows axial view of the lumbar spine with labeling of the anatomical sites, axis line perpendicular to the lamina in the transverse plane, and axis line perpendicular to the skin;
Fig. 7 shows the lateral view of several adjacent spinal segments, axis line perpendicular and parallel to the lamina in the sagittal plane, axis line perpendicular to the skin, and the different access path using conventional devices and methods and the novel advantageous access paths of the present embodiment;
Fig. 8 shows a sagittal view of the patient with parallel and perpendicular axis lines of the lamine, and axis perpendicular to the skin in the different regions of cervical, thoracic, and lumbar spine;
Fig. 9 shows the lateral view of several adjacent spinal segments with embodiments of surgical devices according to embodiments of the invention accessing the lamina nearly parallel to the lamina's longitudinal axis with a smaller diameter cannula, with the tissue tip nearly conforming to the tissue being treated, and for comparison purposes also showing a conventional large diameter cannula accessing the lamina essentially perpendicular to the skin and laminar surfaces;
Fig. 10 shows an embodiment of a surgical device according to the invention accessing lamina through a curved path with the cannula tissue tip approximately parallel to the longitudinal axis of the lamina, and for comparison purposes also showing a conventional large diameter cannula accessing the lamina essentially perpendicular to the skin and laminar surfaces;

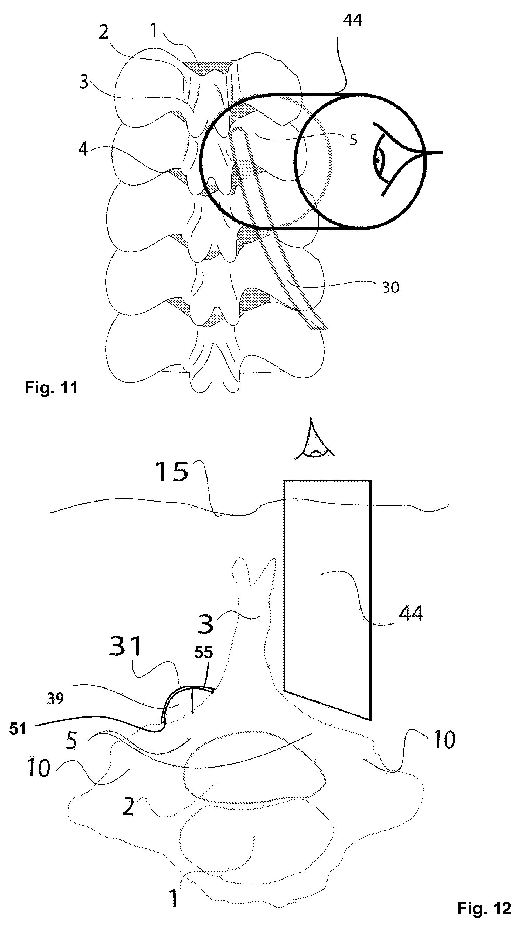

Fig. 11 shows a conventional direct visualization technique of gaining access to the posterior spine were the lamina is being treated using a conventional cannula inserted into the body approximately perpendicular to the skin or laminar surface with the cannula insertion point in the skin being approximately over the tissue being treated with its line of sight being shown, compared to a novel curved cannula accessing the same lamina through a different path and having the cannula's tissue tip placed approximately parallel to the longitudinal axis of the lamina, the novel cannula being inserted through the skin at a different entry point;
Fig. 12 shows an axial image of the spine illustrating on the right side a variant of a conventional direct visualization technique of gaining access to the lamina, compared to a cannula tissue tip of an embodiment of the invention accessing the lamina illustrated on the left side of the image;
Fig. 13 shows a cannula tissue tip of a surgical device according to an embodiment of the invention;
Fig. 14 (a), (b), (c) shows cannulas of surgical devices according to embodiments of the invention with straight, angled, and curved shafts;
Fig. 15 shows an axial view of an embodiment of the novel cannula over the spines process;
Fig. 16 (a), (b), (c), (d) shows lateral views of different cannula tissue tips of surgical devices according to embodiments of the invention designed to fit on to different tissue structures, the different tissue tips having varying shape and dimensions for fitting on to different tissue structures.
Fig. 17 (a), (b), (c), (d) shows inferior views of the different cannula tissue tips of surgical devices according to embodiments of the invention designed to fit on to different tissue structures, the different tissue tips having varying shape and dimensions for fitting on to different tissue structures, with the option of specific designs for the right and left side of the patient;
Fig. 18 shows cannulas of surgical devices according to embodiments of the invention with multi-curved shafts to allow the same skin incision (i.e. entry into the body) to be used to access left and right side laminas;
Fig. 19 shows a cannula with multi-curved shaft of a surgical device according to an embodiment of the invention being used to gain access to the inferior aspect of the lamina;
Fig. 20 shows a surgical device according to an embodiment of the invention in different positions, whereby the tissue tip is moved from one lamina after operation to a second lamina for operation and possible further successive laminas, using the same skin incision (i.e. entry into the body); with either the same cannula or a cannula of different geometry.
Fig. 21 shows a variation of the embodiment to access the under surface of the lamina, and to remove the ligamentum flavum and other soft tissue structures.
Fig. 22 and 23 shows a surgical device according to an embodiment of the invention with a tissue tip that resets on the lamina superior to the lamina being treated and being used as a fulcrum to elevate the lamina being treated after it has been cut;
Figs. 24a, 24b and 25 show a surgical device according to an embodiment of the invention with a straight cannula with a straight trochar that can be removed, fig 24b showing the surgical device provided with a handle;
Figs. 26 and 27 show a surgical device according to an embodiment of the invention with a curved trochar that can be removed;
Fig. 28 (a), (b), (c), (d) shows cross-section views of the different cannulas of surgical devices according to embodiments of the invention;
Fig. 29 (a), (b), (c) shows cannulas of surgical devices according to embodiments of the invention with straight, angled, and curved shafts with guiding systems within and along the cannula;
Fig. 30 (a), (b), (c), (d) shows cross-section views of the different cannulas of surgical devices according to embodiments of the invention with some of the possible options for placement of the internal guiding system in the lumen of the cannula on either side;
Fig. 31 shows a perspective view of a possible configuration of the cannula with an an internal guiding system in the lumen of the cannula;
Fig 32 shows the configuration of figure 31 with a tool inserted in the cannula;
Fig 33 shows a front cross-sectional view of a variant of the cannula with an internal guiding system in the lumen of the cannula;
Fig 34 shows a perspective cross-sectional view of a tissue tip of the cannula of figure 33;
Fig. 35 shows the configuration of figure 33 with a tool inserted in the cannula;
Fig. 36 shows the configuration of figure 34 with a tool inserted in the cannula;
Fig 37 shows a front cross-sectional view of another variant of the cannula with an internal guiding system in the lumen of the cannula;
Fig 38 shows a perspective cross-sectional view of a tissue tip of the cannula of figure 37;
Fig. 39 shows the configuration of figure 37 with a tool inserted in the cannula;
Fig. 40 shows the configuration of figure 38 with a tool inserted in the cannula;
Fig. 41 shows an axial view of the prone patient within an imaging device demonstrating a method in which the present invention can be used;
Fig. 42 shows a surgical device according to the invention assembled on an operating table;
Fig. 43 shows a side view of the prone patient within an imaging device demonstrating a method in which the present invention can be used, the surgical device shown lined up with the patient's cervical spine;
Fig. 44 is similar to figure 43 but showing the surgical device lined up with the patient's the patient's lumbar spine;
Figs. 45 and 46 show the sagittal section of the patient with a surgical device according to the invention being inserted approximately parallel to the lamina being treated, showing a central trochar inserted in the cannula (fig. 45) and being removed as the tissue tip is set into the operating position (fig. 46);
Figs. 47 and 48 shows the sagittal section of the patient with the surgical device after having been inserted as shown in figures 45 and 46, with a cutting device being inserted through the cannula (fig. 47) and cutting through the lamina (fig. 48);
Fig. 49 shows a cross section of a cannula tissue tip of a surgical device according to the invention, partially surrounding the lamina being treated with cutting device configured to partially cut through the one side of the lamina to form a hinge;
Fig. 50 shows the posterior view of a patient's spine demonstrating the ability of a surgical device according to the invention to approach the spine from different directions;
Fig 51 shows a sagittal CT reconstruction of a surgical device according to the invention positioned on the lamina;
Fig. 52 shows a CT axial view of a vertebrae with the a surgical device according to the invention in an operating position;
Fig. 53 shows a sagittal CT reconstruction of a surgical device according to the invention positioned on the lamina, showing a cutting device (burr) inserted through the cannula;
Fig. 54 shows the sagittal CT view of a prior art cannula with significant artifact and posterior shadowing; and
Fig. 55 shows an axial CT view of the prior art cannula.

### Detailed description of embodiments of the invention

One of the applications for which a surgical device according to the invention is particularly well adapted is for operating on the posterior structures of the vertebral body, such as for cutting the lamina. The device according to the invention may however be used on various tissues including bone, cartilage, ligaments and tendons for a variety of surgical operations including cutting, filing, cauterizing, and inserting implants or other materials, or for diagnostic operations such as collecting sample tissue (biopsies). The embodiments disclosed should not be interpreted, or otherwise used as limiting the scope of the disclosure. Additionally one skilled in the art will understand that the following description has broad applications, and the discussion of any embodiment is meant only to be exemplary of that embodiment, and not intended to intimate the scope of the disclosure.

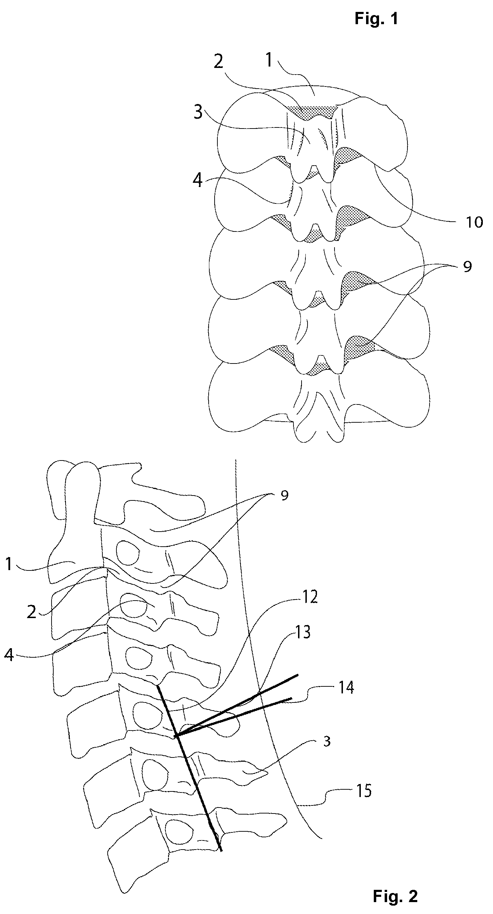

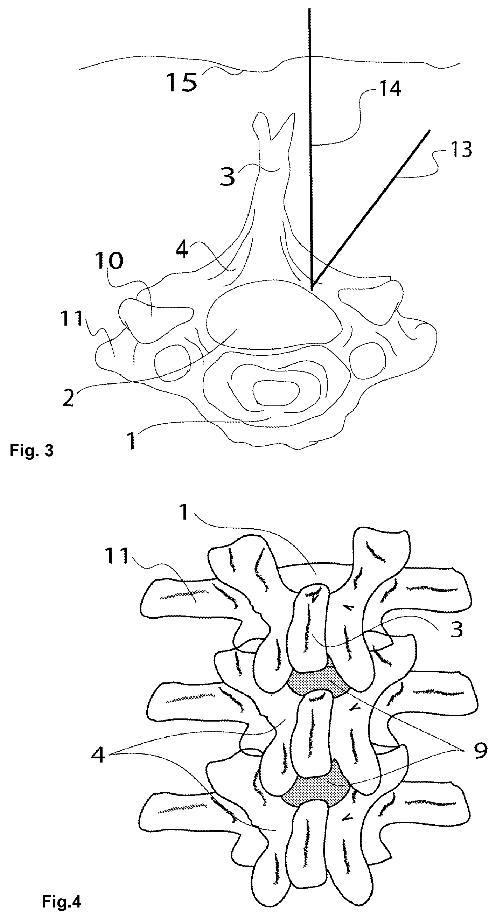

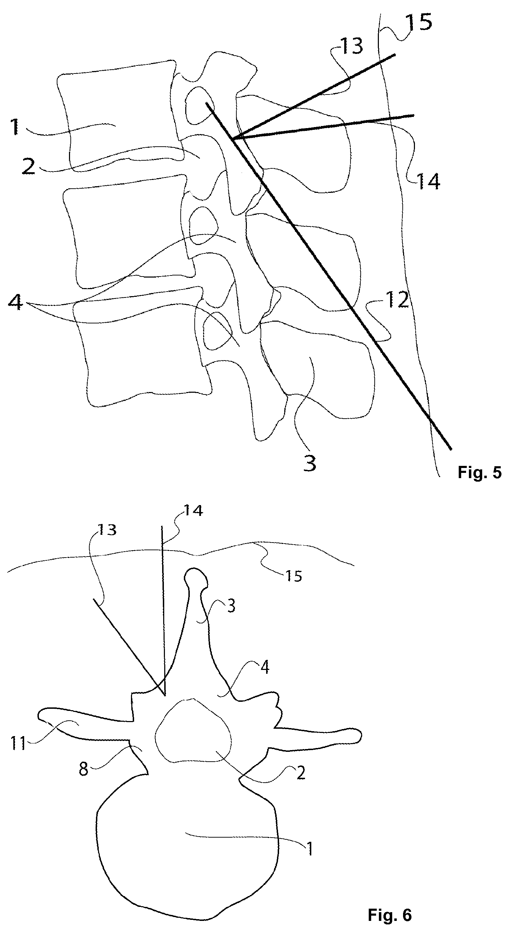

For a better understanding of one of the applications for which the surgical device is well adapted, we shall first briefly describe the relevant anatomy and tissue structures of the vertebral body referring in particular to figures 1-6. Fig. 1 illustrates the posterior view of the cervical spine, Fig. 2 a lateral view of the cervical spine and Fig. 3 an axial view of the cervical spine, showing the vertebral body 1, transverse processes 11, facet joints 10, spinous process 3, lamina 4 on either side reaching from the spinous process 3 to lateral facet joints 10, and a central canal 2 surrounded by the skeletal system. The interlaminar space 9 is the area between two adjacent vertebrae, occupied by the ligament flavum. A surgical instrument introduced in the interlaminar space 9 can rest against the lamina 4 or facet joint 10. The longitudinal axis 12 of the lamina is essentially parallel to the lamina and represents one of the approach directions used by a surgical device to operate on the lamina 4 according to an embodiment of the invention, whereas the axis perpendicular 13 to the lamina and the axis perpendicular 14 to the skin 15 represent paths used by prior art devices and methods for operating on the lamina.

Figures 4, 5, and 6 show the posterior, lateral, and axial views of the lumbar spine showing similar anatomical features as those of the cervical spine, as indicated with the same reference numbers as those used above in relation to the cervical spine.. The thoracic spine is not drawn as it is similar in anatomical make up to regions described for the lumbar and cervical spine.

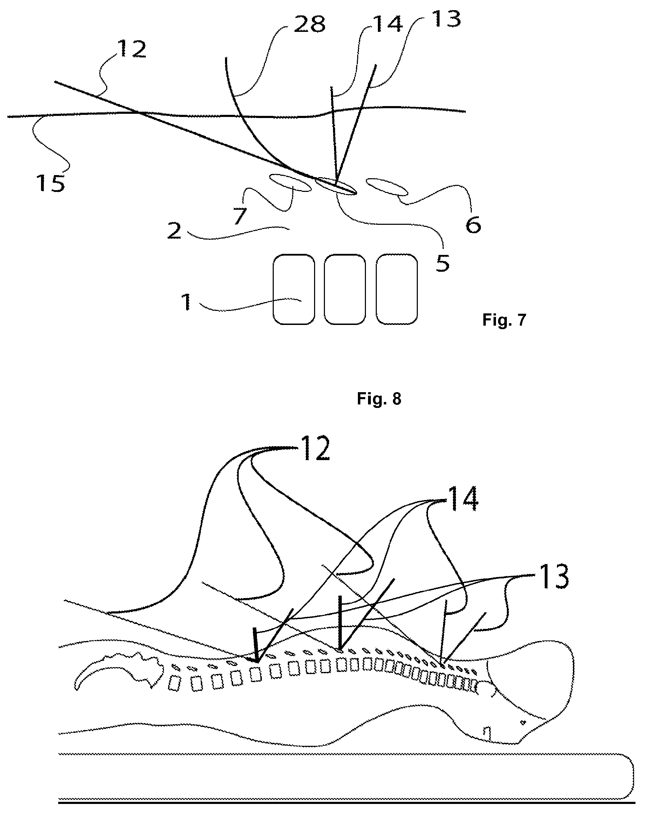

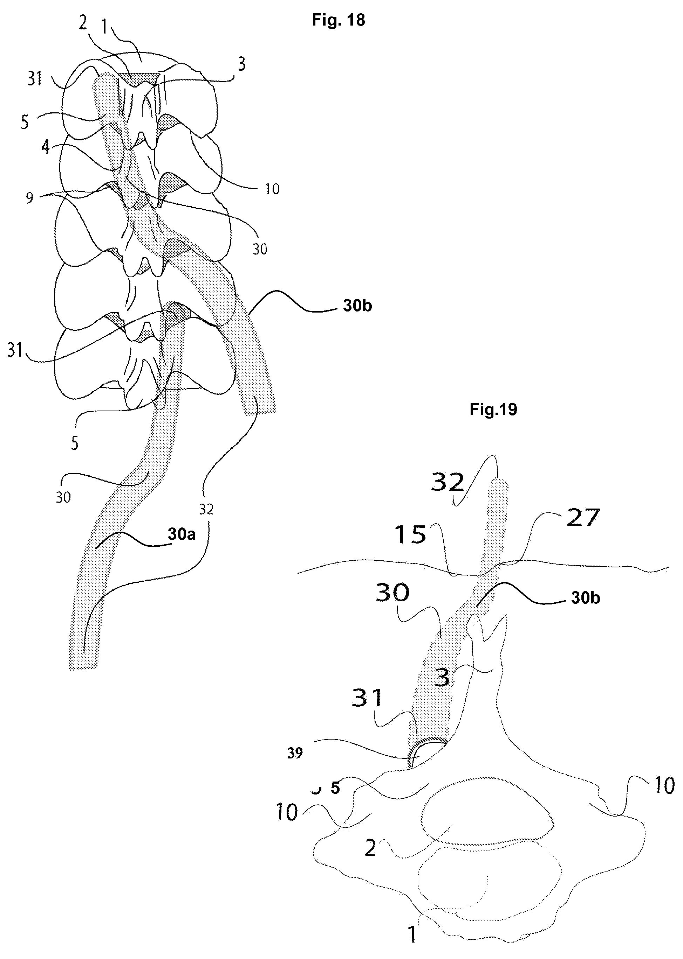

Figures 7 and 8 show a saggital view of the spine with multiple adjacent spinal segments, where the lamina being treated 5 is situated between the lamina superior 6, and the lamina inferior 7 to it. The central canal 2, veterbral bodies 1 and skin 15 are also schematically illustrated. The longitudinal axis of the lamina 12 is one of the paths used by straight cannula according to embodiments of the invention. A curved trajectory 28 is used by curved or angled cannula according to other embodiments of the invention. The axis 13 perpendicular to the lamina and the axis 14 perpendicular to the skin are also illustrated to show the paths used in prior art methods and better explain an important difference between the invention and the prior art. The cannula may be bent in two different planes (as shown in figures 18 and 19) to provide curve paths that are adapted for accessing either the left side or right side of the body.

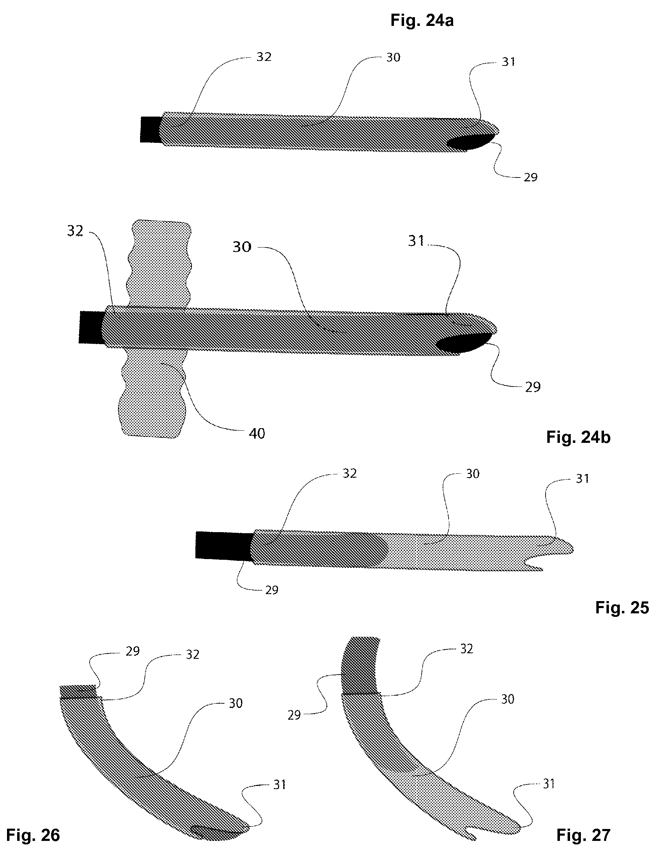

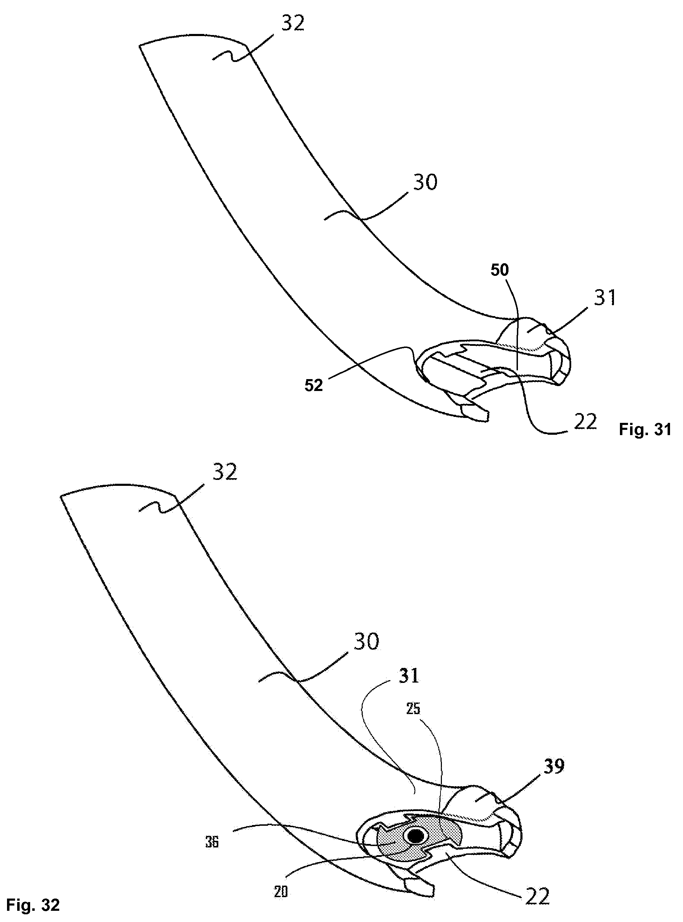

Referring to the figures, in particular figures 24 to 40, a surgical device according to the invention comprises a cannula 30 with a generally tubular wall 50 extending from an access end 32 to a tissue tip 31 and surrounding at least one lumen 52, and one or more instruments 20, insertable in to the lumen of the cannula. In certain embodiments, depending on the applications, the one or more instruments are removably insertable in the lumen 52 of the cannula through the access end 32, and insertable to the tissue tip 31. Within the scope of the invention, depending on the application and the operation to be performed, it is also possible to provide an instrument received in the lumen 52 of the cannula that is non-removably mounted in the lumen. In other applications, the surgical device may be configured to receive different instruments in the cannula during an operation. Once the cannula is in operating position in a patient, an instrument may thus be inserted through the access end 32 and then removed and exchanged for another instrument to perform different operations without removing the cannula from the patient.

The surgical instruments may include surgical instruments configured for cutting, sawing, burring, drilling, shaving, ablating, performing ultrasonic osteotomes, and devices involving cutting and removal of bone and soft tissue structures. The surgical device's access end 32 can also comprise a fitting (not shown) configured to be coupled to a syringe or other delivery mechanisms for injectable substances such as anesthetic substances, medication, filing or binding substances (cements) and implants.

The cannula may be provided with more than one lumen, for example by having an internal partitioning wall (not shown) in the cannula or by having one or more pipes or tubes (not shown) positioned within the lumen 52. The different lumens may each be configured for insertion of different surgical instruments, or used for aspiration of the surgical site or for injection of liquid, or for insertion of a camera.

The surgical device according to the invention is configured to be used with a medical imaging system, such as CT (computed tomography), MRI (magnetic resonance imaging), ultrasound, X-ray, fluoroscopy or other scanning systems for image guidance. In this regard, the material properties and dimensions of the cannula are configured to minimize imaging artifact to provide an operably useful view of the tissue to be treated and the surgical instrument. In particular, the cannula wall is configured to allow partial transparency with respect to the scanning signal such that at least portions of the instrument inserted in the cannula are visible to allow the relative position of the instrument to the tissue to be treated to be accurately controlled. The diameter of the cannula can be minimized to the required size for insertion and guidance of the surgical instruments to the surgical site without additional requirements such as line-of-sight visual access, whereby the small cannula cross-section also significantly reduces imaging artifact compared to conventional surgical devices. Cannula of the surgical device according to the invention may advantageously have cross sectional outer dimensions (*H*, *W*) ranging from approximately 4mm to 12mm.
In certain embodiments, the thickness (*T*) of the cannula wall in at least one scanning direction, in units of centimeters multiplied by the device material's density in units of grams per cubic cm, ranges in value between 0.05 and 0.8 grams per centimeter squared, preferably between 0.1 and 0.4 grams per centimeter squared. The latter advantageously allows partial transparency (i.e. passage of) x-ray beams and magnetic fields through the cannula in at least one direction to allow substantial visualization of anatomical structures, and surgical instruments and implantable medical devices in the cannula lumen and adjacent to the cannula without substantial imaging artifact.

Another advantageous feature of the surgical device that minimizes imaging artifact at the interface between the cannula tissue tip 31 and the tissue to be operated (i.e. the surgical operation site), is the shape of the tissue tip configured to conform to the shape of the tissue against which the tissue tip is set. The tissue tip thus substantially conforms to the structure being treated and reduces air and fluids at the interface between the cannula and the surgical site of the tissue to be operated.

The cannula may advantageously have a substantially smooth and geometrically symmetrical cross section which further assists in minimizing creation of imaging artifacts, resulting from density differences between the surgical device and surrounding tissues.
The geometric and material properties may further be optimized based on the type of imaging system utilized. The cannula may comprise or be made of various biomedical compatible materials that are also compatible for use with the medical imaging system, including ceramics, peek, polymers, carbon fiber, and titanium alloys.

In a particular embodiment for surgical intervention on the vertebral system, the surgical device may advantageously include an electrical stimulator (not shown), which for instance may be in the form of an electrode positioned on the cannula at the tissue tip 31, or on an electrode positioned in the lumen 52 in the region of the tissue tip, to continuously monitor in real-time the position of the surgical device relative to a nerve or neurological structures. In this embodiment the cannula and the instrument in the lumen may comprise portions of insulating material in order to be insulated from conducting electricity from the electrical stimulator.

The above mentioned medical imaging systems are not limitative of the invention, whereby the surgical device according to this invention may be configured to be used with various other medical image guidance systems, including x-ray , fluoroscopy, ultrasound, MRI, CT, flat panel, navigation and camera.

The surgeon using the surgical device thus controls the operation via an electronic imaging interface, rather than by direct visual view of the surgical site as effected for example in prior art surgical methods for operating on the vertebral system. Prior art cannula and MIS devices for operating the vertebral system have a larger cannula 44, for instance at least 18 mm diameter and greater, that are only described and diagramed as being straight, with intention of allowing the operator or a camera system to look down the cannula so that the operator can visualize directly the area being treated. The advantage of using CT, MRI, flat panel, ultrasound, fluoroscopy, navigation, or other medical image guidance apparatus to perform the desired procedure, removes the limitation of the prior art of needing a larger cannula to allow both visualization of the tissue structures being treated, and also space for the insertion of the surgical equipment used for the treatment. Advantageously the herein described invention allows the operator to safely use a smaller diameter cannula with cross sectional dimensions ranging from approximately 4mm to 12mm, with its inherent advantage of causing less trauma to the surrounding tissue.

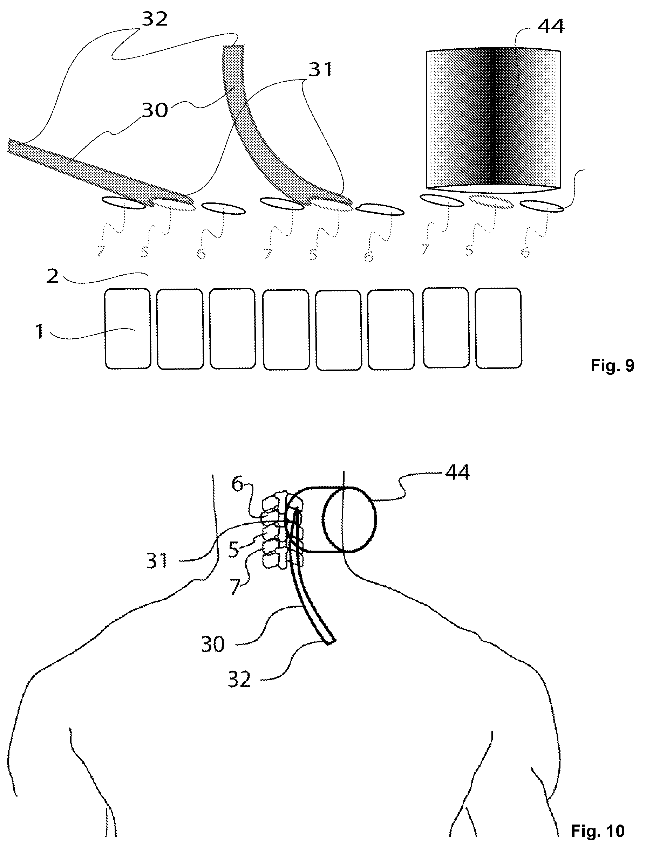

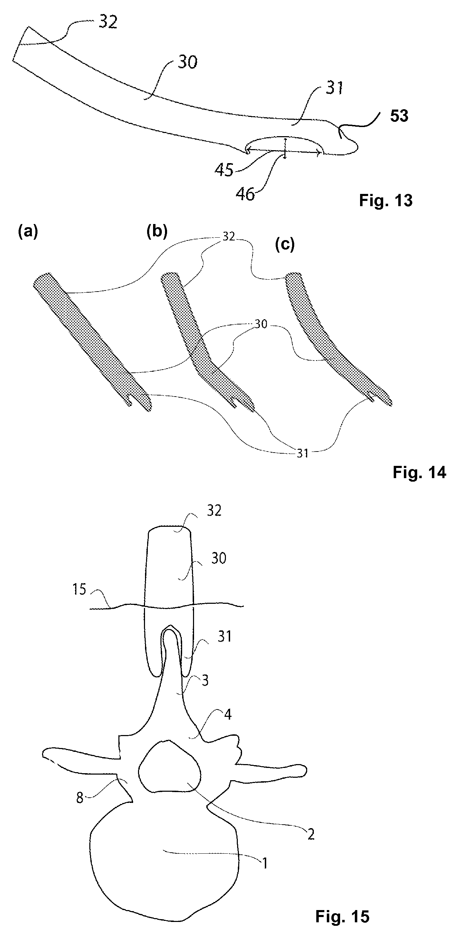

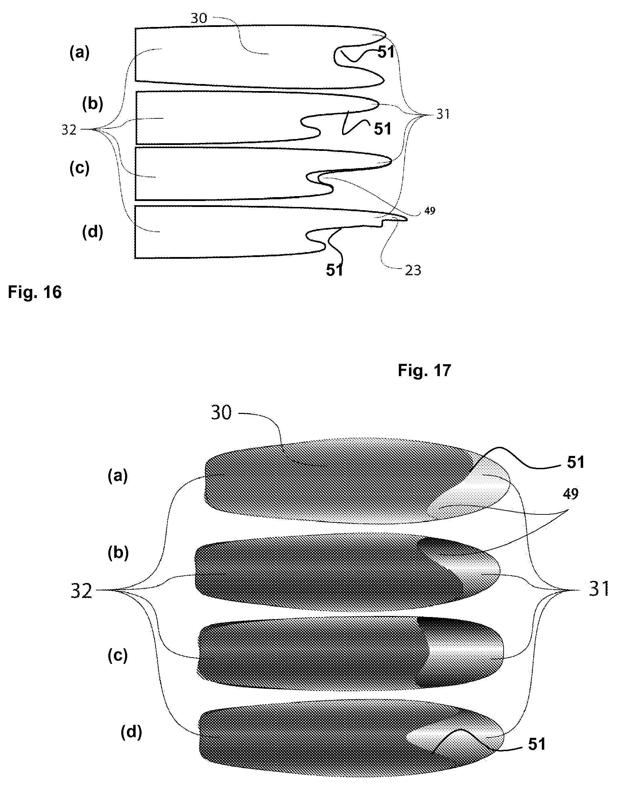

The cannula has a non-straight shape with one (cf. fig. 14b, c; fig. 20-23; fig. 26, 27; figs. 31-40) or more (cf. fig. 18, 19) bends or curves in order to shorten or optimize the path to the surgical site while enabling the tissue tip to be optimally positioned for operation with respect to the tissue to be operated. An optimized path reduces trauma to tissue, and an optimal positioning of the tissue tip enables easier and/or safer operation of the tissue to be operated as well as reducing the invasiveness of the surgical operation. The tissue tip 31 is advantageously contoured so that it has edges 51 configured to rest on the tissue adjacent to the tissue being treated. The resting edge 51 of the tissue tip may be at the apex of the tip of the cannula that is inserted into the body (as shown for example in the embodiments of figures 9, 14, 15, 20, 21-24). Alternatively the edges of the tissue tip can be positioned at a given distance behind the tip of the cannula that is inserted into the body behind a cap at the apex of the cannula 53 of the cannula as shown in the embodiment of figure 13. In the latter variant, the cap at the apex of the cannula 53 may serve as a guide for surgical instruments inserted in the lumen, or serve as a reservoir for debris generated during the surgical intervention. Various tissue tip designs are possible to allow substantial fitting of said tissue tip on to and adjacent to single and multiple spinal lamina, spinous process, spinal facet joints, vertebral body, ribs, sacrum, pelvis, and segments of the skeletal system.
The resting edges 51 of tissue tips in certain embodiments may have a non-symmetrical contour, for instance as shown in figures 16 and 17 configured differently for left and right sides of tissue structures such as for accessing the right and left side of the vertebral system. This configuration is in part achieved by having different geometries for the right and left edges.

The geometry of the resting edges 51 of the tissue tip may advantageously be used to control the depth, thickness, length, and area of the tissue being treated by instruments inserted through the lumen.

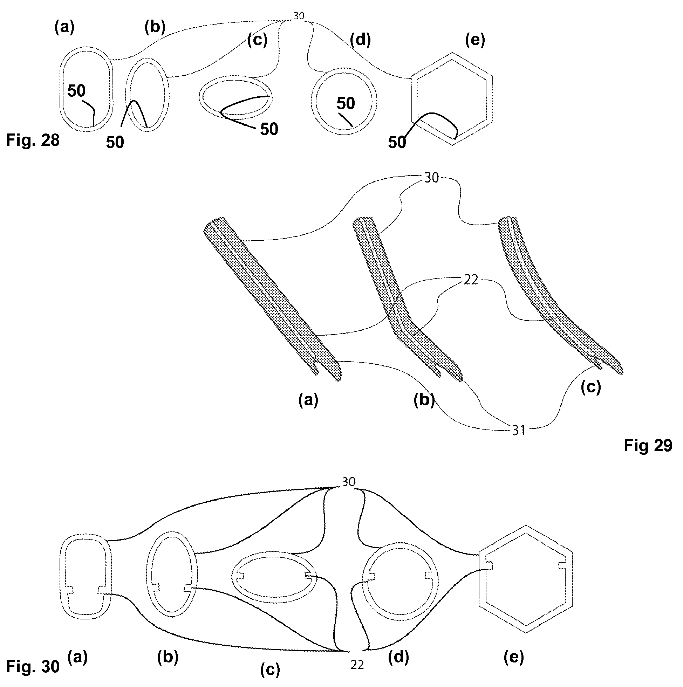

The wall 50 of the cannula may have various cross-sectional profiles, including oval, elliptical, circular, square, rectangular, hexagonal, octagonal, and variable sided geometric cross sections as shown in figures 28 and 30.

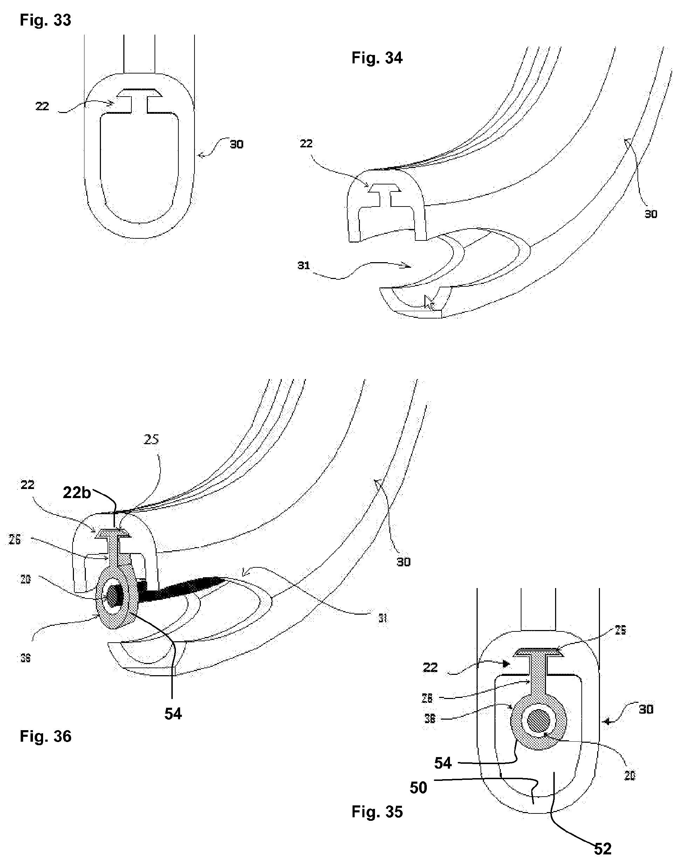

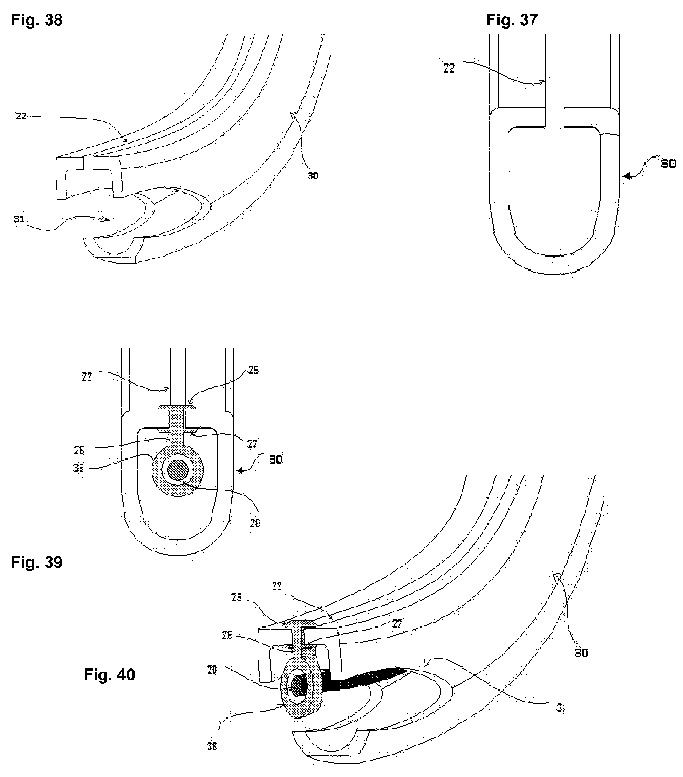

One or more guide elements 22 are formed in the cannula wall 50 for guiding the instrument inserted in the cannula. The guide elements may be in the form of one or more railing protrusions 22a (cf. figs. 30-32) or grooves 22b (cf. figs. 33-36) or a slit (cf. figs. 37-40), all extending in the longitudinal direction of the lumen. The instrument may be provided with one or more complementary guide elements 36, that may be configured not only to allow sliding insertion of the instrument through the cannula, but also act as a bearing to support rotating instruments (e.g. as illustrated in figures 39, 40) such as instruments with burrs. A plurality of guide elements 36 may be positioned along the instrument shaft at intervals there along. In certain embodiments, the cannula may not have any particular guide rails or grooves, although the instrument may be provided with guide elements or spacers engaging the inner surface of the cannula wall 50 so as to position the instrument within the lumen. The guide elements advantageously also ensure that the instrument extremity inserted into the tissue tip 31 remains stably guided and extending in the required direction so that the depth of the cutting, burring or other operation is well controlled. The difference in size and the path taken by the prior art cannula 44, and the cannula 30 described in the present embodiments in either the curved or straight configuration is shown in Fig 9. The conformity of the tissue tip 31 to the lamina is shown. A direct posterior view of the patient with the patient's head is on top portion of the image is shown in Fig. 10. The prior art cannula 44 is shown as well as the cannula 30 of an embodiment positioned over the lamina being treated 5, where the tissue tip 31 is resting upon the lamina that is being treated 5. The larger size of the prior art cannula 44 as well as the need for the line of sight, as compared to the invention cannula 30 become readily apparent, as well as the different trajectories for reaching the lamina being treated 5. A posterior view of the spine with the prior art cannula 44, and a curved cannula 30 according to an embodiment of the invention is shown in Fig 11. Both systems are applied to the same lamina being treated 5, to further demonstrate their different paths. The smaller diameter of the cannula 30 of the present invention can be also be appreciated, when compared to the prior art cannula 44. When viewing the vertebra from an axial cross section as in Fig. 12, the differences between the prior art cannula 44, and invention cannula 30 with its tissue tip 31 over the lamina being treated 5, becomes more apparent. The prior art cannula 44 is introduced in a substantial vertical manner with its longitudinal access approximately perpendicular to the laminar surface, whereas the tissue tip 31 of an embodiment of the invention has its longitudinal axis L essentially parallel to the lamina's longitudinal axis. This causes minimal disruption to the soft tissues overlaying the lamina as opposed to the direct vertical approach taken by the prior art cannula 44.

A sloped tip design of a variant of the prior art cannula 44 is shown in Fig. 12 that allows for some surface accommodation to the bone; however it has a constant slope. In an embodiment of the invention, the tissue tip 31 resting edge 51 has a curved cross-sectional profile 55 which takes into account the three dimensional shape of the tissue being treated, in this example the lamina 5 of a vertebra.

An important advantage of embodiments of the cannula is the tissue tip 31 that substantially conforms to the tissue being treated which is shown for instance in figures 12 and 13. This is achieved by having a length 45, and depth 46 of the tissue tip 31 that is configured for the tissue shape and size to be operated. The variability of the tissue tip 31 is a novel and advantageous feature with respect to the prior art. As shown in Fig. 14, the surgical device includes the option of the cannula being straight (a), angled (b), or curved (c). Other embodiments include placing the cannula over other skeletal structures such as the spinous process 3 shown in Fig. 15. Some of the various options for different tissue tips allowing for conformation to the tissue being treated is demonstrated in Fig. 16 and Fig. 17, showing the lateral (fig. 16) and inferior (fig. 17) views of these embodiments of tissue tips. These variable tissue tips 31 permit the cannula of an embodiment of the inventions to substantially conform to the tissue being treated in three-dimensions. Additionally different designs for accessing the left and right side of the body are shown, with asymmetrical resting edges 51a, 51b.

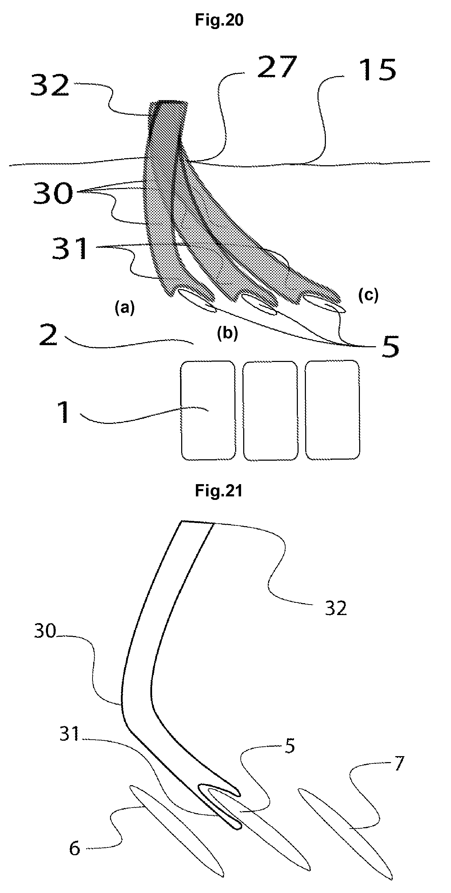

The use of a cannula 30 curved in two planes with specific left 30a and right sided 30b cannulas is illustrated in Fig. 18 with the posterior cervical view and Fig. 19 with an axial view of the vertebra. This allows treatment of both the left and right side through a single midline skin incision, and aids the operator to position the tissue tip 31 in exactly the desired position, on the lamina being treated 5. A surgeon can thus treat multiple adjacent lamina or other tissues through a single skin incision. This advantage is further illustrated by the embodiments shown in Fig. 20 where curved cannula 30 with different curvatures (c), (d), (e) are used to allow the tissue tip 31 to reach different lamina through a single skin entry point 27.

Fig. 21. illustrates a variation of the embodiment to access the under surface of the lamina, and to remove the ligamentum flavum and other soft tissue structures.

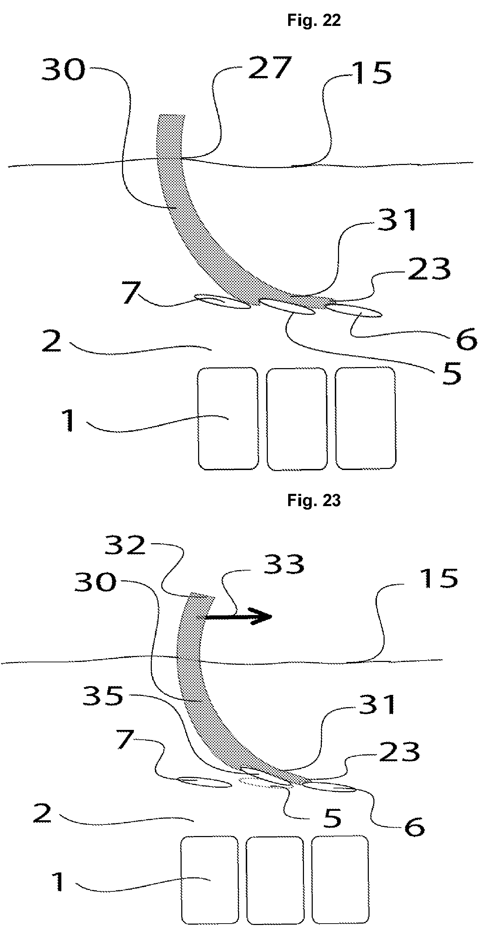

Fig. 22 and 23 illustrates another embodiment of a cannula 30 with a tissue tip 31 comprising a fulcrum 23 configured to rest on the spinal lamina 6 superior to the one being treated 5. The operator can thus use the cannula 30 as lever to move and manipulate the lamina being treated 5. The lamina inferior to the one being treated 7 is also shown to allow for better understanding of the figure. The force 33 applied to the cannula 30 can for example be used to elevate the segment 35 of the lamina 5 after it has been divided.

In Figs. 24 to 27 a removable trochar 29 is shown inserted in the cannula 30. The trochar 29 allows the straight curved or angled cannula 30 to be inserted more easily through soft tissue to reach the tissue structures being treated. The trochar 29 also prevents the entering of unwanted tissue into the cannula as it is being advanced.

The cannula 30 as shown in Figs. 28, 30, 33 and 37 may be provided with different cross sections depending on the surgical procedure to be performed and/or the configuration of the instruments for insertion in the cannula.

As shown in Fig. 29-39, a further optional feature of embodiments of the invention is the provision of a guiding system 22 extending longitudinally along the inside of the cannula 30. This guiding system in the form of guide rail or slot or slit 22 formed along the cannula wall 50 and a complementary instrument guide element 26 mounted on the instrument 20 inserted in the cannula lumen 52 allows surgical tools and implantable medical devices to travel down the cannula 30 in a guided fashion, following the trajectory of the cannula. The instrument guide element 26 comprises a sliding portion 25, 27 that slides within the grove, slot, or railing 22, of the cannula 30, and a bearing or bushing portion 36 to hold rotatably or fixedly the surgical instrument 20. The instrument guide element 26 helps keep the instrument 20 in a defined position within the cannula 30. This helps the operator to introduce surgical tools 20 and implants with great precision and accuracy, especially when used in conjunction with medical image guidance.

As shown in figure 24b, the cannula 30 can further be fitted with a handle 40 to allow the user to have better control over the cannula. The handle 40 can be placed at the access end 32 of the cannula 30. The handle 40 can be placed such that it does not interfere with the passage of the central trochar 29, or surgical instruments and medical devices that are passed through the cannula 30.

In one aspect of the present invention, the surgical device is configured for intervention to the posterior vertebral column. The operator identifies a section of at least one area of interest. The cannula is inserted and stabilized by resting against its tissue tip 31 on the skeletal system. This is performed under image guidance, without substantially dissecting the paravertebral muscles. Alternatively the laminar surfaces could be cleared of soft and bone tissues creating greater space.

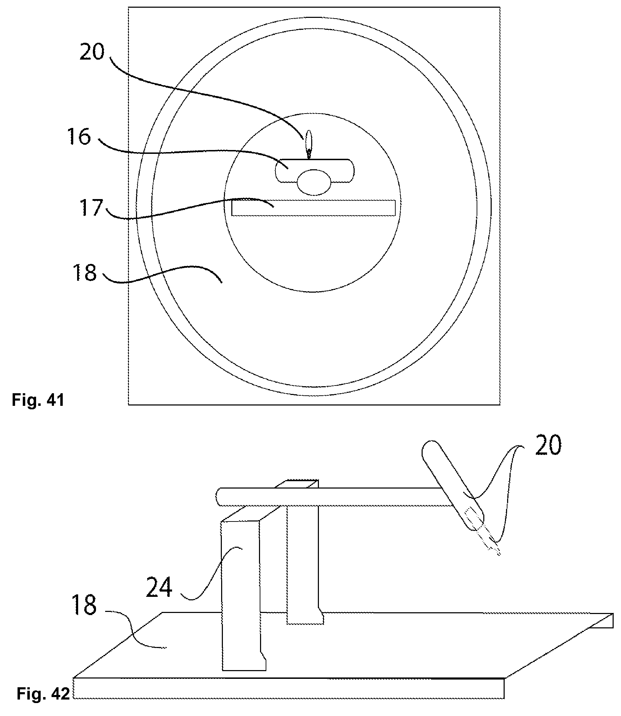

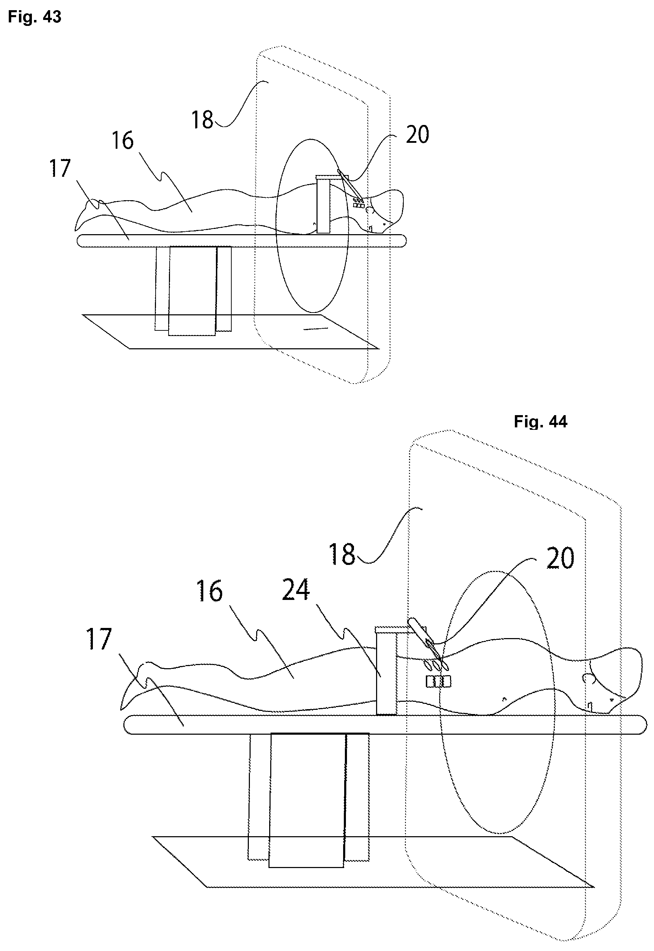

The patient 16 is positioned prone on the procedure table 17 as shown in Fig 41. Sometimes, the patient may have to be placed in a lateral decubitus, semi-prone or semi-supine position, to ensure patient comfort and to minimize patient movement. The use of a head rest or tongs may be needed to assist in patient positioning. MRI, CT, image guidance, or fluoroscopy 18 is performed to localize the working area. Following preliminary imaging scan, using the most appropriate images, a path is selected that is the most ideal route for directing the cannula to the surgical site being treated. The cannula can be positioned on a stand as illustrated on Fig 42. Fig 43 and 44 respectively show the posterior cervical and lumbar regions being treated, respectively.

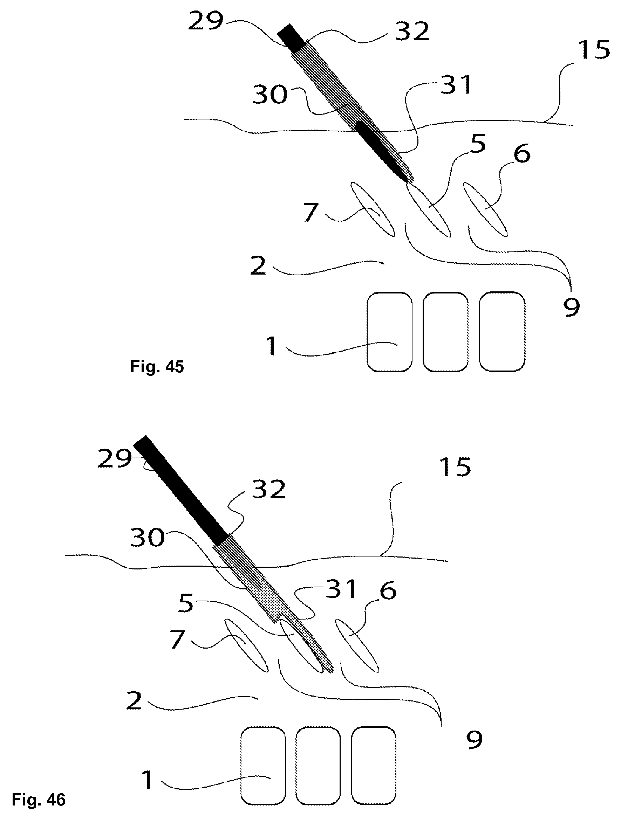

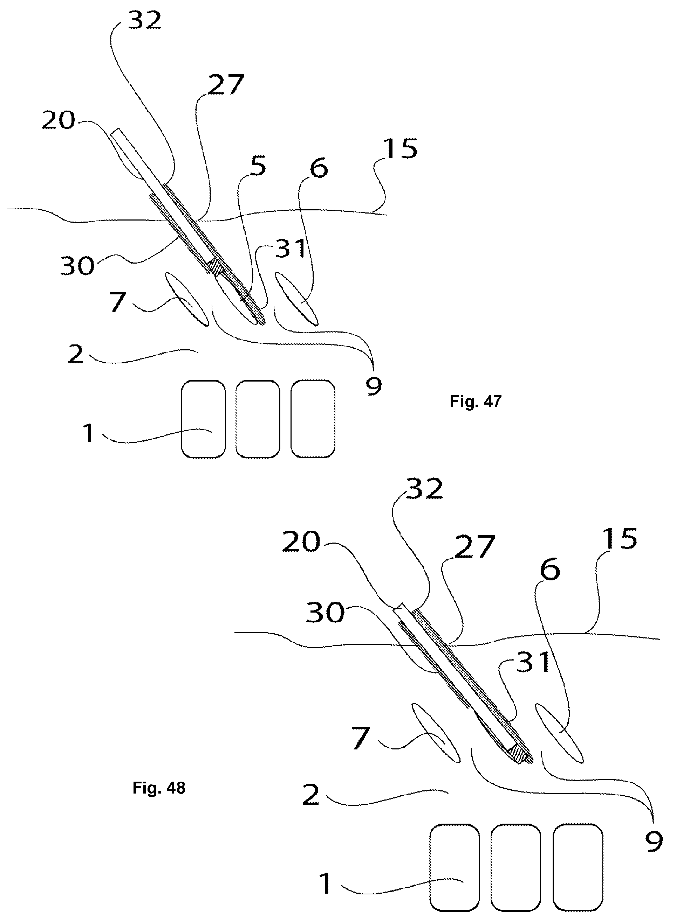

The entry point at the patient's skin is calculated based on the preliminary imaging scan and the insertion procedure is carried out step by step as shown in Fig 45-48 as follows:
- after administration of local anesthesia, an incision at the skin entry point 27 is made,
- the surgical device comprising a trochar 29 fully inserted in the cannula 30 (fig. 45) is advanced up to the tissue 5 (lamina in this instance) to be treated under intermittent or continuous image guidance or navigation
- the trochar is removed and the cannula fully advanced to the operating position where the edge 51 of the tissue tip 31 rests on the tissue to be treated 5 (fig. 46) whereby imaging is performed to ensure proper position of the cannula tissue tip,
- a surgical instrument 20 with a tool (in this instance a burr on a flexible rotor) is inserted in the cannula and slidably advanced to the surgical site, whereby the instrument guide element 26 guides the tool 20 to the operation site (fig. 47), and
- the operation is performed, in this instance by further advancing the tool under intermittent or continuous image guidance or navigation to (in this instance cutting through the lamina with the rotating burr) (fig. 48).

The above steps may also be performed with a curved cannula and with other surgical instruments inserted in the cannula.

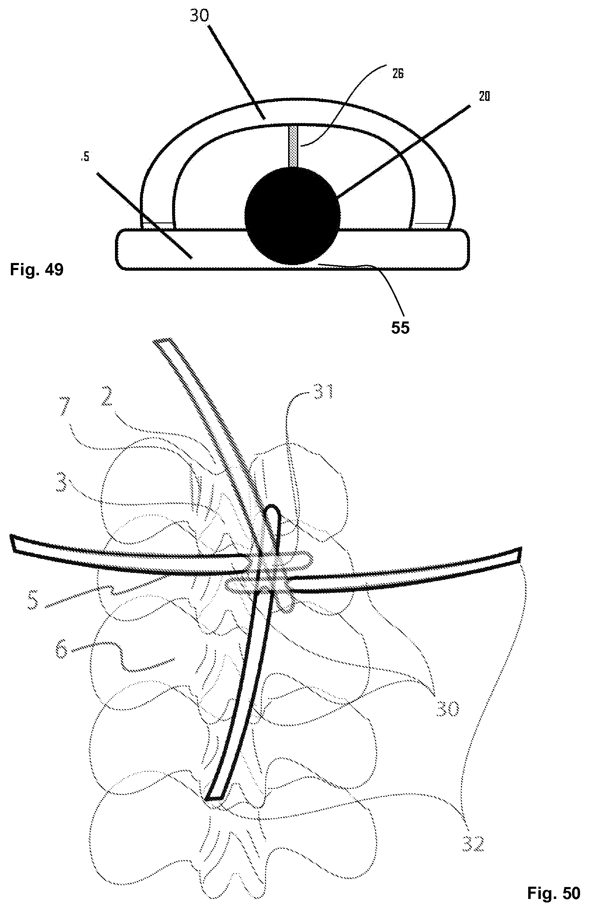

Fig. 49 is an axial view of the lamina 5 being machined by a surgical burr 20. It may be noted that the operation may require the complete sectioning of the tissue 5, or only a partial machining leaving a tissue bridge or hinge portion 15. The invention is particularly advantageous for such operations since the depth of cutting can be well controlled in view of the ability to accurately position the tissue tip on the tissue to be treated and provide a tool insertion/advance direction parallel to the surface of the tissue to be treated. Moreover, advancing the surgical tool parallel to the surface of the tissue to be treated significantly enhances safety of the operation, especially for operations on the vertebrae, in particular on the lamina since this direction is essentially parallel to the spinal column nerve. The invention affords great flexibility in the sense that the tissue tip can be configured to conform to the tissue to be treated and the direction of insertion of the surgical tool in the cannula configured at the desired angle of operation.

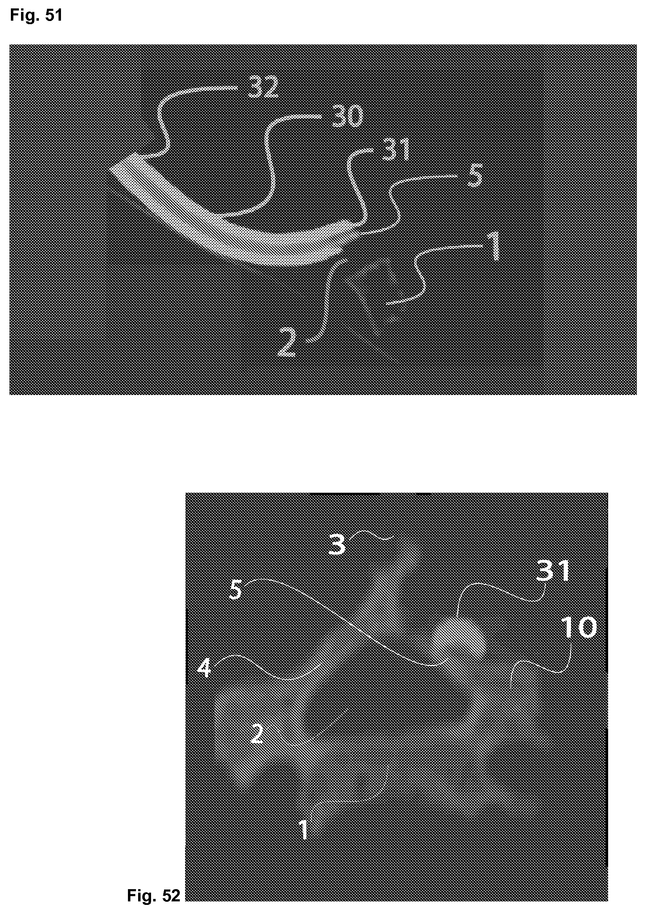

As illustrated in Fig 51 the cannula 30 can be introduced in different directions increasing the ease of use for the operator. After the procedure, the incision site should be closed and dressed.

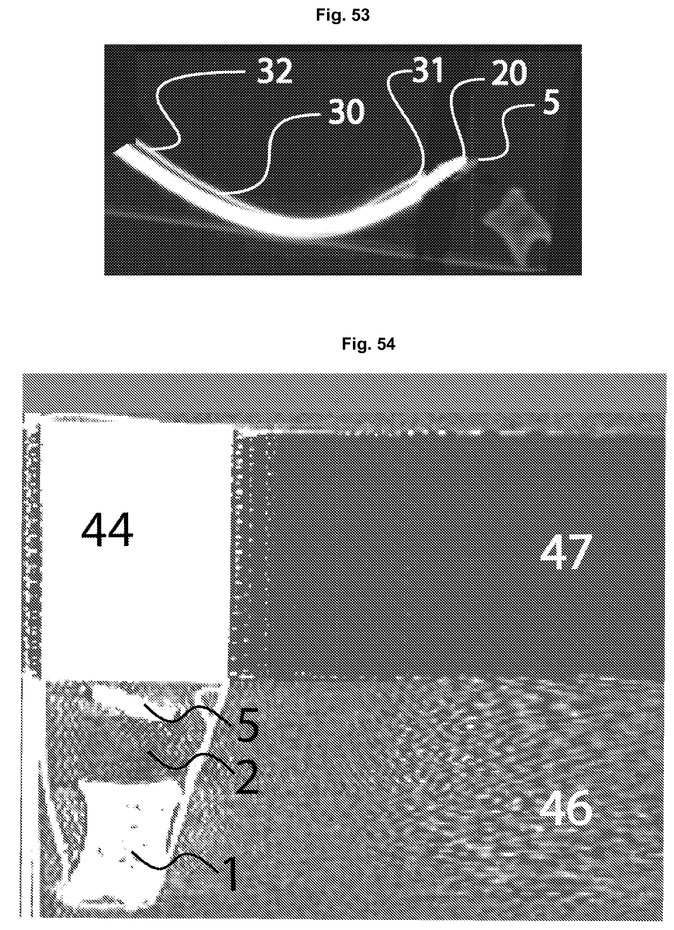

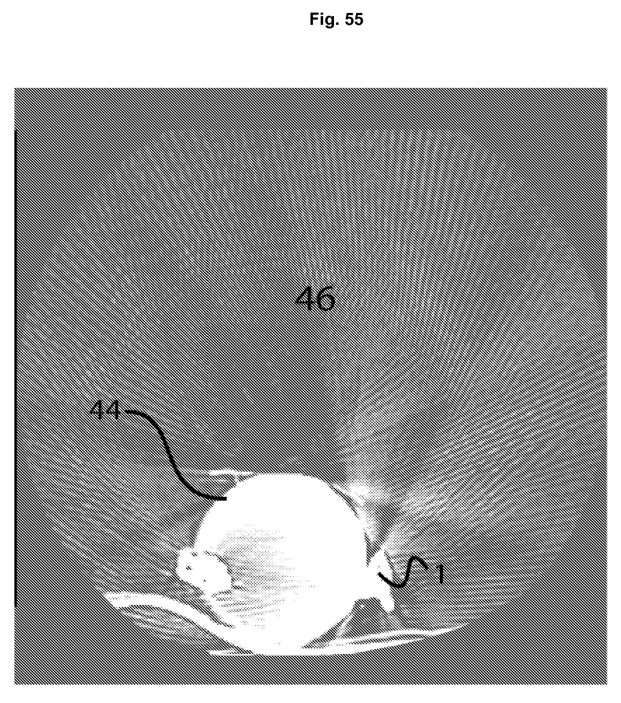

Advantages of the surgical device according to the invention are further facilitated by the cannula's design and material properties allowing the cannula to be partially transparent with minimal artefact with different imaging techniques. Fig. 51, 53 and 52 illustrate a CT scan of a surgical device according to the invention in use on a vertebrae, where figures 51, 52 shows a sagittal view and figure 52 an axial view. The utilization of material and dimensions that allow the cannula to be partially transparent during imagining permits the use of the surgical device for highly precise and accurate cutting, allowing the advancement of the instrument 20 be visualized using imaging while the procedure is being performed as shown in figures 51 and 53. The position and function of the surgical instruments 20 can be monitored at all the times. The advantages of an embodiment of the invention becomes further apparent when compared to the sagittal (Fig 54) and axial view (Fig 55) of the prior art cannula 44, where major imaging artifact 46 and imaging shadow 47 is noted.

## Claims

1. Surgical device for invasive surgery, comprising a cannula (30) with a wall (50) surrounding at least one lumen (52) extending from an access end (32) to a tissue tip (31), and one or more instruments (20, 29) slidably receivable and guidable in the lumen of the cannula, wherein
the surgical device is configured to be used with a medical imaging system for image guidance of the device, the cannula wall being configured to allow partial transparency with respect to a scanning signal of said medical imaging system such that at least portions of the instrument received in the cannula are visible;
the cannula has a non-straight shape with one or more bends or curves;
**characterised in that** the cannula tissue tip is configured to rest on human spinal facet joints and lamina,
the tissue tip having a curved edge (51) shaped to conform to the shape of the spinal facet joints and lamina such that the cannula is stabilized only by resting its tissue tip on the spinal facet joints and lamina;
one or more guide elements (22) are formed in the cannula wall for guiding the instrument received in the lumen of the cannula as it slides through the lumen of the cannula.

2. Surgical device according to claim 1 wherein a thickness (*T*) of the cannula wall in at least one scanning direction multiplied by a material density of the cannula wall, has a value between 0.05 and 0.8 grams per centimeter squared.

3. Surgical device according to any one of the preceding claims wherein the material of the cannula is selected from a group consisting of ceramics, peek, polymers, carbon fiber, metallic alloys, and titanium alloys.

4. Surgical device according to any one of the preceding claims wherein the edge (51) of the tissue tip is positioned at an extremity of the cannula configured to be inserted into a patient's body.

5. Surgical device according to claim 4 wherein the edge (51) of the tissue tip is positioned adjacent to a cap at an extremity of the cannula configured to be inserted into a patient's body.

6. Surgical device according to any of the preceding claims wherein the edge (51) of the tissue tip is non-symmetrical.

7. Surgical device according to any one of the preceding claims wherein the guide elements are in the form of one or more railing protrusions (22a), grooves (22b) or slits (22c) extending in a longitudinal direction of the lumen.

8. Surgical device according to any one of the preceding claims wherein the instrument comprises one or more instrument guide elements (36) configured to allow sliding insertion of the instrument through the cannula.

9. Surgical device according to claim 8 wherein the instrument guide elements comprise a bearing portion to support and allow rotation of an instrument.

10. Surgical device according to any of the preceding claims wherein the cannula is bent or curved essentially in a single plane.

11. Surgical device according to claim 10 wherein the cannula is bent or curved in at least two planes.

## Patentansprüche

1. Chirurgische Vorrichtung für invasive Chirurgie, aufweisend eine Kanüle (30) mit einer Wand (50), die wenigstens ein Lumen (52), das sich von einem Zugangsende (32) aus zu einer Gewebespitze (31) erstreckt, und ein oder mehrere im Lumen der Kanüle gleitend aufnehmbare und lenkbar angeordnete Instrumente (20, 29) umgibt, wobei
die chirurgische Vorrichtung konfiguriert ist für eine Nutzung mit einem medizinischen Bildgebungssystem zur Bildführung der Vorrichtung, die Kanülenwand konfiguriert ist, um eine teilweise Transparenz im Hinblick auf ein Abtastsignal des medizinischen Bildgebungssystems auf solche Weise zu gestatten, dass wenigstens Teile des in der Kanüle empfangenen Instruments sichtbar sind;
die Kanüle eine ungerade Form mit einer oder mehreren Biegungen oder Krümmungen aufweist;
**dadurch gekennzeichnet, dass** die Gewebespitze der Kanüle konfiguriert ist für ein Aufliegen auf menschlichen Wirbelgelenken und Lamina,
die Gewebespitze eine gekrümmte Kante (51) aufweist, die geformt ist, um mit der Form der Wirbelgelenke und Lamina auf solche Weise übereinzustimmen, dass die Kanüle allein dadurch stabilisiert wird, dass ihre Gewebespitze auf den Wirbelgelenken und Lamina aufliegt;
ein oder mehrere Führungselemente (22) in der Kanülenwand zum Führen des im Lumen der Kanüle aufgenommenen Instruments geformt sind, wenn dieses durch das Lumen der Kanüle gleitet.

2. Chirurgische Vorrichtung nach Anspruch 1, wobei eine Dicke (T) der Kanülenwand in wenigstens einer Abtastrichtung multipliziert mit einer Materialdichte der Kanülenwand einen Wert von zwischen 0,05 und 0,8 g/cm² aufweist.

3. Chirurgische Vorrichtung nach einem der vorhergehenden Ansprüche, wobei der Werkstoff der Kanüle ausgewählt ist aus einer Gruppe bestehend aus Keramik, Polyetheretherketon (PEEK), Polymeren, Kohlefaser, Metalllegierungen und Titanlegierungen.

4. Chirurgische Vorrichtung nach einem der vorhergehenden Ansprüche, wobei die Kante (51) der Gewebespitze an einem Ende der Kanüle positioniert ist, die für ein Einführen in den Körper eines Patienten konfiguriert ist.

5. Chirurgische Vorrichtung nach Anspruch 4, wobei die Kante (51) der Gewebespitze nahe einer Kappe an einem Ende der Kanüle positioniert und für ein Einführen in den Körper eines Patienten konfiguriert ist.

6. Chirurgische Vorrichtung nach einem der vorhergehenden Ansprüche, wobei die Kante (51) der Gewebespitze asymmetrisch ist.

7. Chirurgische Vorrichtung nach einem der vorhergehenden Ansprüche, wobei die Führungselemente die Form eines bzw. einer oder mehrerer schienenähnlicher Vorsprünge (22a), Nuten (22b) oder Schlitze (22c) aufweisen, die sich in einer Längsrichtung des Lumens erstrecken.

8. Chirurgische Vorrichtung nach einem der vorhergehenden Ansprüche, wobei das Instrument ein oder mehrere Instrumentenführungselemente (36) aufweist, die konfiguriert sind, um ein gleitendes Einführen des Instruments durch die Kanüle zu gestatten.

9. Chirurgische Vorrichtung nach Anspruch 8, wobei die Instrumentenführungselemente einen Lagerbereich aufweisen, um ein Rotation eines Instruments zu stützen und zu gestatten.

10. Chirurgische Vorrichtung nach einem der vorhergehenden Ansprüche, wobei die Kanüle im Wesentlichen in einer Einzelebene gebogen oder gekrümmt ist.

11. Chirurgische Vorrichtung nach Anspruch 10, wobei die Kanüle in wenigstens zwei Ebenen gebogen oder gekrümmt ist.

## Revendications

1. Dispositif chirurgical pour une chirurgie invasive, comprenant une canule (30) avec une paroi (50) entourant au moins une lumière (52) s'étendant à partir d'une extrémité d'accès (32) jusqu'à une pointe de tissu (31), et un ou plusieurs instruments (20, 29) pouvant être reçus et guidés de manière coulissante dans la lumière de la canule, dans lequel
le dispositif chirurgical est configuré pour être utilisé avec un système d'imagerie médicale pour le guidage par l'image du dispositif, la paroi de canule étant configurée pour permettre la transparence partielle par rapport à un signal de balayage dudit système d'imagerie médicale de sorte qu'au moins certaines parties de l'instrument reçu dans la canule sont visibles ;
la canule a une forme non droite avec un ou plusieurs coudes ou courbures ;
**caractérisé en ce que** la pointe de tissu de canule est configurée pour s'appuyer sur les facettes articulaires vertébrales humaines et la lamina,
la pointe de tissu ayant un bord incurvé (51) formé pour se conformer à la forme des facettes articulaires vertébrales et la lamina de sorte que la canule est stabilisée uniquement en appuyant sa pointe de tissu sur les facettes articulaires vertébrales et la lamina ;
un ou plusieurs éléments de guidage (22) sont formés dans la paroi de canule pour guider l'instrument reçu dans la lumière de la canule au fur et à mesure qu'il coulisse à travers la lumière de la canule.

2. Dispositif chirurgical selon la revendication 1, dans lequel une épaisseur (T) de la paroi de canule au moins dans la direction de balayage, multipliée par une densité de matériau de la paroi de canule, a une valeur comprise entre 0,05 et 0,8 gramme par centimètre carré.

3. Dispositif chirurgical selon l'une quelconque des revendications précédentes, dans lequel le matériau de la canule est choisi dans un groupe comprenant la céramique, le polyétheréthercétone (PEEK), les polymères, les fibres de carbone, les alliages métalliques et les alliages de titane.

4. Dispositif chirurgical selon l'une quelconque des revendications précédentes, dans lequel le bord (51) de la pointe de tissu est positionné à une extrémité de la canule configurée pour être insérée dans le corps d'un patient.

5. Dispositif chirurgical selon la revendication 4, dans lequel le bord (51) de la pointe de tissu est positionné de manière adjacente à un capuchon à une extrémité de la canule configurée pour être insérée dans le corps d'un patient.

6. Dispositif chirurgical selon l'une quelconque des revendications précédentes, dans lequel le bord (51) de la pointe de tissu n'est pas symétrique.

7. Dispositif chirurgical selon l'une quelconque des revendications précédentes, dans lequel les éléments de guidage se présentent sous la forme d'une ou de plusieurs saillies de rampe (22a), rainures (22b) ou fentes (22c) s'étendant dans une direction longitudinale de la lumière.

8. Dispositif chirurgical selon l'une quelconque des revendications précédentes, dans lequel l'instrument comprend un ou plusieurs éléments de guidage d'instrument (36) configurés pour permettre l'insertion coulissante de l'instrument à travers la canule.

9. Dispositif chirurgical selon la revendication 8, dans lequel les éléments de guidage d'instrument comprennent une partie d'appui pour supporter et permettre la rotation d'un instrument.

10. Dispositif chirurgical selon l'une quelconque des revendications précédentes, dans lequel la canule est courbée ou incurvée essentiellement dans un seul plan.

11. Dispositif chirurgical selon la revendication 10, dans lequel la canule est courbée ou incurvée dans au moins deux plans.
